# EUROPEAN PATENT APPLICATION

(11) **EP 2 135 614 A1**
(43) Date of publication of application: **23.12.2009**
(21) Application number: 09002067.8
(22) Date of filing: 13.02.2009
(51) Int. Cl.: A61K 35/74, A61K 36/064, A61K 31/733, A61K 31/721, A61P 1/12, A61P 1/00

(54) **Intestinal dismicrobism compound having anti-diarrhoea, anti-inflammatory and lactose and lactose derivative intolerance lenitive properties**

(30) Priority: 20.06.2008 IT MI20081124
(71) Applicant: EURITALIA S.r.l., 15067 Novi Ligure (AL) (IT)
(72) Inventor: Bonabello, Elvio, 15067 Novi Ligure Alessandria (IT)
(74) Representative: Cicogna, Franco

(57) **Abstract**

The present invention relates to an intestinal dismicrobism compound having anti-diarrhoea, antiinflammatory and lactose and lactose derivative intolerance lenitive properties which comprises a prebiotic matrix, constituted of organic and inorganic nourishing substances, and a probiotic matrix, containing bacillus coagulans bacteric cells.

## Description

### BACKGROUND OF THE INVENTION

The present invention relates to an intestinal dismicrobism compound having anti-diarrhoea, anti-inflammatory and lactose and lactose derivative intolerance lenitive properties.

As is known, it has been practically found that in the human intestinal bacterial flora, an important symbiosis equilibrium between the human body, and intestinal bacterial flora occurs so as to provide a mutual advantageous condition.

Thus, for some strains, the activity of which has been found to be particularly important, the term "probiotic" has been specifically coined, just to underline the positive contribution to an improved wellbeing of the host organism.

It has been further found that some non assimilable food compounds favorably affect colonies of the intestinal bacteric flora, in particular selectively stimulate their growth and reproduction, and have accordingly been called "prebiotic" substances.

A mixing of probiotic and prebiotic substances provides compounds which are called "symbiotic", which are prepared or made on making industrial chain thereon processes such as lyophilizing, storing and transporting processes which render survival of cells selected for formulating the above products very difficult are performed.

In particular, the lyophilizing process constitutes the weakest link of such a making chain, since the dehydrating process of the substance being lyophilized involves a high bacterium death rate, sometimes up to 90% of the cells; moreover, even if a comparatively high number of surviving cells could return to a vital condition at an intestinal level they, however, would be damaged at their DNA or RNA level, thereby loosing their reproduction capability and limiting their actual beneficial contribution to a simple temporary activity ending at their death time.

Another great problem to be overcome in formulating the above products, is that the above bacteria ideal life environment is of a basic type and, to provide the desired prebiotic activity at an intestinal level, the cells must also pass through the digestive tube gastric portion, the high acidic condition of which constitutes another frequent cell death source.

All the above making difficulties have as a consequence that, notwithstanding the beneficial effect of bacteric flora on the human body, not only on the intestinal mucosa, which has been scientifically clearly demonstrated, many gastroenterologists think that an administration of milk enzymes or ferments would be usually useless, since only the host organism bacteric flora would be able of self multiplying to properly repopulate the mucosa.

To try to solve the above mentioned problem, products containing only prebiotic-action substances, such as inuline, fructo-oligosaccharides or beer yeast substances have been presently introduced into the market, which substances, however, for economic saving reasons do not include probiotic bacteria.

### SUMMARY OF THE INVENTION

Accordingly, the aim of the present invention is to overcome the above mentioned problems by providing a compound having an intestinal bacteric flora rebalancing or re-equilibrating activity.

Within the scope of the above mentioned aim, a main object of the invention is to provide such a compound adapted to hinder growth and development of pathogenic intestinal bacteria causing diarrhoea conditions in a patient, to greatly prevent dismicrobism derived from antibiotic therapies and greatly reduce symptoms of lactose or lactose derivative intolerance.

Another object of the present invention is to provide such a compound adapted to provide a probiotic action for the host organism, thereby efficiently preventing vaginite or vulvovaginite diseases, caused by a colon-genital bacterial translocation in women sensible to such a pathology.

Yet another important object of the present invention is to provide such a compound for rebalancing or re-equilibrating intestinal microflora, which does not cause any cellular death event due to making processes, does not require any controlled temperature storing or transporting operations, and which, upon administering to a patient, is not subjected to possible depletions during a gastric transit because of its acid pH and which, moreover, is adapted to start its probiotic function immediately as it reaches at a patient duodenum.

Yet another object of the present invention is to provide such an intestinal dismicrobism compound which, owing to its specifically designed features, is very reliable and safe in operation.

Yet another object of the present invention is to provide such a compound which is very competitive from a mere economic standpoint.

The above mentioned aim and objects, as well as yet other objects, which will become more apparent hereinafter, are achieved by an intestinal dismicrobism compound having anti-diarrhoea, anti-inflammatory and lactose and lactose derivative intolerance lenitive properties, **characterized in that** said compound comprises a prebiotic matrix constituted of organic or inorganic nourishing substances and a probiotic matrix containing bacillus coagulans bacteric cells.

Further characteristics and advantages of the present invention will become more apparent hereinafter from the following detailed disclosure of a preferred, though not exclusive, embodiment thereof, which will be disclosed in a more detailed manner hereinafter in the following disclosure.

In particular, the intestinal dismicrobism compound having anti-diarrhoea, anti-inflammatory and lactose and lactose derivative intolerance lenitive properties is prepared by using a prebiotic matrix consisting of organic and inorganic nourishing substances.

Said prebiotic matrix is made, in particular, by using non assimilable substances, such as inuline, yeasts, maltodextrins and oligofructosaccharides, since the latter cannot be digested and provide a slight but very useful gastric protection for the bacteric cells.

Differently from a human body, these intestinal bacteric cells are suitable to derive nourishing from the above substances.

Moreover, lyophilized saccharomices cereviseae cells are also.included.

While the above yeast substances do not colonize human intestinal mucosa, they have a very strong metabolic activity with a related production of amino-acids, vitamins and enzymes, providing a nourishing sublayer adapted to greatly accelerate the growth and reproduction of bacteria present at a mucosa level.

The above component is included in a rate variable from 1 to 50% based on the total weight of the daily administering dose.

A very important feature of the prebiotic matrix according to the invention is that it comprises a liquid component including sterile sea water, taken, according to official pharmacopea rules, at a preset sea depth.

As is known, sea water contains dissolved therein all mineral salts and oligoelements which, through thousands years have originated early biologic formations, such as virus, moulds and bacteria.

Thus, since intestinal flora comprises many of the above bacterium species, it should be evident that an enrichment of the sublayer generated by saccharomycetes, which is of a mainly organic nature, with a liquid component fully constituted by inorganic elements, would allow to make a complete culture broth useful not only for intestinal bacteric flora cells, but also for said saccharomycetes.

Consequently, by the above mentioned composition or compound, a prebiotic matrix suitable to provide nourishing not only for intestinal cells but also for yeast cells allowing them to benefit from an extended survival, can be achieved.

More specifically, sea water to be used for achieving that goal must be taken at a sea region characterized by a very low polluting substance presences, such as at a coast structure rich in cliffs and rocks with a sheer drop to the sea, and with a good oxygenation level and a luxuriant algae growth.

According to the invention, the above mentioned compound is used at such a dilution which, with respect to body fluids, may be either isotonic, hypertonic, or hypotonic.

The second component of the invention is a probiotic matrix designed for providing a re-equilibrating activity on bacterial intestinal flora, and for hindering growth and development of pathogenic bacteria, which are symptoms of diarrhoeic conditions, while hindering dismicrobism caused by antibiotic therapies, and mitigating symptoms of lactose and lactose derivate intolerance and which, moreover, is also adapted to provide a probiotic action to the host organism, so as to greatly hinder vaginites and vulvovaginites due to a colo-vaginal bacterial translocation.

In this connection, it should be pointed out that a main feature of the invention is that a specifically designed bacterial cell type, that is bacillus coagulans, is introduced into the above mentioned probiotic matrix.

The provision of said bacillus coagulans is such as not to be subjected to any cellular death effects due to making processes and, moreover, said bacillus coagulans, as it reaches a subject duodenum, provides its probiotic functions thereby producing, in particular, beta-galactosidasis enzymes, allowing to resolve or split lactose to produce lactic acid to mitigate lactose intolerance levels, while simultaneously producing hydrolasis enzymes for deconjugating biliar acids.

Another main feature of the invention is that the inventive compound produces antibiotic-like substances (such as bacteriocines and proteic complex arrangements having a bactericide or bacteriostatic activity), and the hydroxy-methylglutarate enzyme COA, for inhibiting cholesterol formation.

Moreover, the inventive compounds also prevent potentially pathogenic bacteria, due a territorial competition and a local acidifying action.

More specifically, the above mentioned bacillum coagulans produces lactic acid and antibacteric substances, able of deconjugating biliar acids and consequently degrading nitrosamines and also providing good anticancer activities.

Moreover, lactobacillus sporogens increase the adhesiveness to intestinal epithelia, intestinal settling and colonization, a cholesterol blood absorption reduction and increase immune defenses.

In fact, as sporogenic bacteria are removed from their natural environment, they, instead of incurring in a cellular death, produce a capsule just called "spore" therewithin they axe able of surviving even under poor conditions, while always remaining in a quiescent status.

Lab tests carried out on the above bacteric strains have shown a probiotic type of activity, suggesting their use both at a human and a veterinary level.

Moreover, the use of a sporogenic type of fermenting cell such as bacillus coagulans, provides the advantages of a negligible cell loss due to lyophilizing, since these spores are resistant under most extreme temperature and moisture conditions, do not require controlled temperature transporting or storing provisions, and have a vitality and survival much greater than those of conventional lactic enzymes or ferments.

Furthermore, they are not affected by the stomach acid pH but, at a gastric level, they pass from a quiescent status to a germinating condition, thereby immediately starting to colonize duodenum.

Advantageously, said bacillus coagulans is used in an amount varying from 100 millions and 100 thousand millions of vital cells.

For additionally improving the inventive probiotic matrix, a further bacteric strain, that is streptococcus termophilus, is introduced thereinto.

The above bacteric strain is characterized by a good resistance to a conjugated lyophilizing as well as a satisfactory gastric resistance.

The above further bacteric strain is also used in an amount varying from 100 millions to 100 thousand millions of vital lyophilized cells.

Thus, it has been found that, by using the above disclosed compounds as inventively formulated, the organic component of saccharomices cereviseae, associated with the inorganic components comprising sea water dissolved mineral salts and ions, provides the two mentioned probiotic bacteric strains with a much greater vitality than that of conventional lactic enzymes or ferments, thereby providing a much greater efficiency and longer stability.

Moreover, since the invention has been specifically designed for preventing and treating diarroheic conditions, it would be also useful to use therein vitamins of the B group and, more specifically, B1, B2 and B6 vitamins, together with PP vitamins.

According to the invention, the amount of each said vitamin may vary from a minimum of 50% of a daily rational dose to 100% of the latter.

The administering way of the inventive compound, which is used in liquid form, provides to use small bottles with a separated metering plug, containing the lyophilized substances, or mixed contents vial elements.

It has been found that the efficacy of lactic ferments, which is still subjected to accurate controls, has overcome the 18° test month, without significant losses thereof.

From the above disclosure it should be apparent that the invention fully achieves the intended aim and objects.

In particular, the fact is to be pointed out that the invention provides a compound based on a use of a particular type of cells, which, since they produce a spore, can hold a vital condition up to a patient administering expiring date, independently from the manner the invention has been made and preserved.

The above mentioned cells, moreover, are suitable to pass through the acid environment of the digestive apparatus, without qualitative or quantitative modifications, thereby assuring a stable and long duration colonization of intestinal mucosa.

The inventive compound is made in a liquid form, and accordingly is very suitable for a pediatric age administration.

Moreover, the inventive compound can further comprise fruit and meant aromatizing substances, or any other suitable aroma.

In this connection it should be moreover pointed out that the liquid component, that is sea water, does not represent a mere excipient but, due to the presence therein of dissolved mineral salts and ions, constitutes a true and proper active principle.

The invention, as disclosed, is susceptible to several modifications and variations, all of which will come within the scope of the invention.

Moreover, all the details can be replaced by other technical equivalent elements.

## Claims

1. An intestinal dismicrobism compound having anti-diarrohea, anti-inflammatory and lactose and lactose derivative intolerance lenitive properties, **characterized in that** said compound comprises a prebiotic matrix including organic and inorganic nourishing substances and a probiotic matrix including bacillus coagulans bacterial cells.

2. A compound, according to claim 1, **characterized in that** said prebiotic matrix comprises lyophilized saccharomices cereviseae cells.

3. A compound, according to the preceding claim, **characterized in that** said organic nourishing substances comprise inuline, yeasts, maltodextrins and oligofructosaccharides.

4. A compound, according to one or more of the preceding claims, **characterized in that** said prebiotic matrix comprises a liquid component.

5. A compound, according to one or more of the preceding claims, **characterized in that** said liquid component comprises sterile sea water.

6. A compound, according to one or more of the preceding claims, **characterized in that** said bacillus coagulans is present in said compound in an amount varying from 100 millions to 100 thousand millions of vital cells.

7. A compound, according to one or more of the preceding claims, **characterized in that** said probiotic matrix comprises a streptococcus termophilus bacteric strain.

8. A compound, according to one or more of the preceding claims, **characterized in that** said streptococcus termophilus is present in said compound in an amount variable from 100 millions to 100 thousand millions of lyophilized vital cells.

9. A compound, according to one or more of the preceding claims, **characterized in that** said compound comprises B group vitamins.

10. An intestinal dismicrobism compound having anti-diarrohea, anti-inflammatory and lactose and lactose derivative intolerance lenitive properties, according to the preceding claims and as broadly disclosed and illustrated for the intended aim and objects.
